# EUROPEAN PATENT SPECIFICATION

(11) **EP 1 427 476 B1**
(45) Date of publication and mention of the grant of the patent: **25.03.2009**
(21) Application number: 02776884.5
(22) Date of filing: 06.09.2002
(51) Int. Cl.: A61N 1/36

(54) **APPARATUS FOR STIMULATING A BODILY HOLLOW SYSTEM AND FOR MEASURING REACTIONS TO STIMULI OF SUCH SYSTEM**
BALLONKATHETERSYSTEM ZUR STIMULATION UND MESSUNG DER DURCH STIMULI ERZEUGTEN REAKTION
APPAREIL POUR STIMULER UN SYSTEME CREUX DU CORPS ET POUR MESURER LES REACTIONS DUDIT SYSTEME AUX STIMULI

(30) Priority: 06.09.2001 DK 200101301
(43) Date of publication of application: 16.06.2004
(73) Proprietor: Gregersen, Hans, 8543 Hornslet (DK)
(72) Inventor: Gregersen, Hans, 8543 Hornslet (DK)
(74) Representative: Plougmann & Vingtoft A/S
(86) International application number: PCT/DK2002/000581
(87) International publication number: WO 2003/020124

(56) References cited:
- WO-A-00/48672
- WO-A-01/24721
- WO-A-99/64104
- US-A- 4 502 490
- US-A- 4 809 710
- US-A- 5 275 169
- US-A- 5 423 742
- US-A- 5 682 899
- US-B1- 6 277 136

## Description

### FIELD OF THE INVENTION

The present invention relates to an apparatus for stimulating walls of bodily hollow systems, and also for measuring sensational or physical reactions towards such stimulation.

### BACKGROUND OF THE INVENTION

Visceral pain is among the most common form of pain experienced in medical practice. Despite its greater clinical importance, visceral pain is less well understood and treated than pain from the skin and other somatic tissues This is mainly due to the difficulties of characterizing the pain, typically described in terms of unpleasant and diffuse sensations with autonomic reactions such as nausea and sweating. In the clinic fear, anxiety and cognitive reactions may also blur the observation of pain as part of an illness. Finally, visceral pain is often part of a multi-organ syndrome with systemic reactions such as fever and malaisc.

To overcome such problems, experimental pain assessment models have been developed to explore pain mechanisms. Most experimental studies in the gut have been performed in anesthetized animals, with the nociceptive response based on neurophysiological or behavioral reactions. As human pain is a multi-modal experience composed of sensory, psychological and physiological aspects, results from animal experiments can, however, only partly be extrapolated to man. Hence, several human models have been developed to stimulate somatic structures. In such models the investigator can control the stimulus with respect to localization, intensity and duration. The response can be assessed quantitatively and qualitatively . However, only few models for visceral experimental pain stimuli exist, mainly due to the difficulties for complicated testing equipment to come into contact with the internal organs. The major limitation of the existing human models is that they may not mimic clinical pain, as they are based on single, short-lasting stimuli only partly involving the many mechanisms typically activated during diseases Thus, the needs for experimental visceral models mimicking more closely the clinical situation are needed. Such a model should be based on multi-modal testing regimes where different receptors and central nervous system mechanisms are activated.

US 4,809,710 describes a catheter for making pressure measurements in a sphincter canal. The catheter is provided with an Inflation tube and water-perfusion tubes. A balloon or bag is inflatable through the inflation tube. The water-perfusion tubes have side openings communicating with the sphincter canal. The catheter is capable of providing a three-dimensional pressure profile in a sphincter canal by sensing and measuring canal pressures simultaneously at a plurality of sensing points located In one or more straight lines parallel to an axis and at predetermined levels. The apparatus is only capable of sensing and measuring pressures, and is neither not capable of providing any kind of stimulation to the sphincter canal. The advantage of the catheter described in this piece of prior art is that It provides the possibility of measuring pressures simultaneously at different levels, however, the pressure being provided by the sphincter muscles. Thus, the catheter is only sensing the pressure along an extension of catheter above the balloon, and the balloon is only intended for fixing the catheter in relation to the sphincter canal.

US 5,513,639 describes another type of catheter, also comprising an inflatable balloon member attached to and free of a tubular support member. An array of elongated piezo-electric elements is arranged inside the balloon member. Space is provided between the elements and the balloon for ensuring a space In which the array of piezo-electric elements can rotate irrespective of the shape of the balloon member. The balloon member can be Inflated by Introducing a liquid therein. The balloon member is Inflated subsequent to the introduction of the catheter into the esophagus. The array of piezo-electric elements are used for ultrasonic scanning of in body cavities and is capable of scanning the pain inflicted on the patient during insertion of the probe into a body cavity. Thus, it is the introduction of the probe, which is measured, and not a certain stimulation being subjected subsequent to the insertion of the probe. Once the probe is inserted into the body cavity and while the diagnosis is carried out, the patient does not feel pain, thus no stimulation is taking place after the insertion of the probe. Furthermore, only ultrasonic scanning is capable of being performed by the catheter. Also, the balloon is only intended for fixing the catheter In reflation to the esophagus.

US 4,502,490 describes a method of monitoring the depth of anaesthesia of a patient by using an inflatable oesophageal balloon. It is disclosed that signals indicative of oesophageal contractions is obtained by use of a sensor. From the signal, an output indicative of the rate of occurrence is derived. Also an apparatus for carrying out the method is disclosed.

The apparatus comprises a catheter being provided with an inflatable balloon situated between a proximal end and a distal end of the catheter, means for passing an inflating fluid. Preferably a liquid, from the proximal end to the balloon, and means for introducing a number of the following stimuli into the hollow system: mechanical stimulus, thermal stimulus and electric stimulus, said means for introducing said number of stimuli being provided between the proximal end and the distal end of the catheter. The apparatus being furthermore provided with means measuring at least one of the following physical properties of the balloon subsequently to applying the stimuli: the pressure of fluid inside the balloon and the temperature of fluid inside the balloon.

### SUMMARY OF THE INVENTION

The object of the present invention is to provide an apparatus capable of applying chosen stimuli to the bodily hollow system and subsequently to the stimulation measuring any reaction from a patient after of during the stimulation.

The object according to the invention is obtained by an apparatus for stimulating walls of bodily hollow systems and for measuring a subsequent physical reaction, said apparatus comprising a catheter being provided with an inflatable balloon situated between a proximal end and a distal end of the catheter, the apparatus comprising means for passing an inflating fluid, preferably a liquid, from the proximal end to the balloon, the apparatus furthermore comprising means for introducing a chemical stimulus into the hollow system, said means for introducing the chemical stimulus being positioned between the proximal end and the distal end of the catheter, and the apparatus being provided with means for measuring at least one of the following physical properties of the balloon subsequently to applying the chemical stimulus: the volume of the balloon, the cross-sectional area of the balloon seen in a direction parallel to a longitudinal extension of the bodily hollow system, when the apparatus is Introduced Into the body, the diameter of the balloon in a plane perpendicular to a longitudinal extension of the bodily hollow system, when the apparatus is introduced into the body, the tension of the balloon, the strain of the balloon, the pressure of a fluid inside the balloon, and the temperature of a fluid inside the balloon.

It is important to notice that by the denomination "balloon" is meant only a bag capable of being inflated. The inflation need not result In a dilation of the material of the balloon. Thus, perhaps the balloon is made of a material, which subsequent to inflation is not subjected to any dilation, but merely has an increased volume due to the Inflation. Accordingly, In the remainder of application, apart from In the test results In the last part of the specification, the denomination "balloon" will be used, because this is the commonly used denomination, although the balloon may be as a bag, i.e. no dilation of the bag.

### BRIEF DESCRIPTION OF THE DRAWINGS

The invention will now be described in detail with reference to the drawing, where
fig. 1 Is a sketch showing an apparatus according to the invention being introduced Into the esophagus of a patient for physical stimulation and/or measuring properties,
fig. 2 is a sketch of the apparatus itself being provided with a number of means for stimulating and a number of means for measuring in a bodily hollow system.

### DETAILED DESCRIPTION OF THE INVENTION

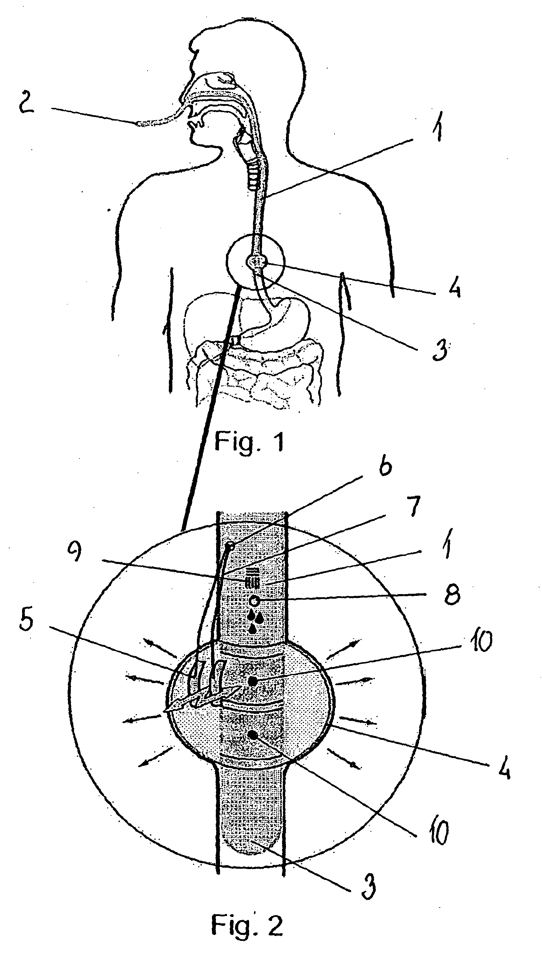

**Fig 1**. shows how an apparatus for providing stimuli and/or for measuring certain properties in a bodily hollow system may be utilised in the esophagus of a patient. The apparatus consists of a catheter, alternatively denoted a probe, having a proximal end 2 and a distal end 3. The distal end 3 is Introduced into a readily accessible opening from the outside of the esophagus, i.e. the mouth or, as shown, the nose and further down into the esophagus towards the stomach. Nearby the distal end 3, the catheter is provided with a balloon 4, also called a bag, which is inflated so that an outer surface of the balloon is abutting the inner surface of the wall of the hollow system, i.e. of the esophagus. The inflation takes place only after the catheter has been introduced into the esophagus. By inflating the balloon, the balloon and thus the catheter is fixed in relation to the esophagus so that the catheter, in the situation shown, cannot be displaced longitudinally along the length of the esophagus.

Preferably, but not necessarily, the catheter Is fixed at the proximal end as well, i.e. is fixed to the nose or any other outer surface or organ. In the situation shown, where the catheter is introduced through the nose, fixation of the proximal end may take place in any suitable manner, perhaps by a clamp being clamped to the wing of the nose, to the nasal bone or to the bridge of the nose. Once introduced Into the bodily hollow system, the apparatus may be used for stimulating the hollow system of a person or an animal by one or more of the stimuli: mechanical stimulus, thermal stimulus, chemical stimulus and electric stimulus. Alternatively, or in addition, the apparatus may be used for measuring a physical reaction of a person or an animal, when the bodily hollow system of the person or the animal is being subjected to a number of artificially applied stimuli of the above-mentioned type.

The balloon of the catheter is preferably made from polyestherurethane being a material readily extendable, being non-harmful to the human or animal body and having distinct physical properties such as elasticity, modus of strain etc. The thickness of the material, which the balloon is made of, is perhaps between 30 µm and 50 µm. However, any other well-suited material may be used as long as it fulfils the need for expandability, non-permeability of the fluid inflating of the balloon, and security towards not being harmful to the body. Thus, polypropylene (PP) or polyethylene (PE) may alternatively be used. Even other materials may be used referring to the fact that the denomination "balloon" need not imply that the material, which the balloon is made of, is dilated, when the balloon, or the bag which it may also be called, is inflated. Thus, materials having much less elasticity than the above-mentioned materials may be used, perhaps materials which, when inflated, does not show any noticeably elastic deformation. Also the thickness of the material, which the balloon is made of, may be thicker than the dimensions mentioned above, thus perhaps also resulting in that the material, when inflated, does not show any noticeably elastic deformation.
The size of the balloon, when inflated, differs depending on which bodily hollow system the apparatus is used in and depending on which part of the system in question, that the apparatus is used in. As example, for use in the esophagus, the balloon normally has a size when being inflated of up to 40 mm in diameter seen in a plane perpendicular to the longitudinal direction of the esophagus. However, abnormalities of the esophagus such as outpouchings may necessitate a balloon having an increased diameter when inflated. Also, other hollow systems have either smaller or larger cross-sectional areas than the esophagus. Thus, the cardiovascular system have much smaller cross-sectional areas, whereas the small intestine and even the large intestine and the rectum normally have larger diameters than the esophagus, perhaps necessitating a balloon having up to 80 mm in diameter when inflated.

In the situation shown, the apparatus shown is a longitudinal catheter being introduced into the esophagus of a human being. However, the apparatus may be used on animals as well. Furthermore, the apparatus may be used in any hollow system of the body of the person or the animal, non-limiting examples of such being: the digestive system including the stomach, preferably the gastrointestinal tract, a part of the urogenital system including the urinary bladder, or part of the cardiovascular system including the heart.

Fig. 2 shows part of the apparatus as the one shown in fig. 1. The part shown is the balloon 4 and the part of the catheter 1 nearby the balloon. Different means for stimulating and for measuring are shown. Firstly, as shown with arrows, the balloon is inflated by means of a fluid, preferably a liquid, and more preferred salt water introduced to the balloon through a canal provided in the catheter. The fluid is pumped to the balloon from an exterior reservoir (not shown) such a sterile bag containing the fluid, the pumping being provided by, as example, a pump with rollers exerting a pumping action on a hose, and the fluid Is pumped from the reservoir to the proximal end 3 of the catheter. The pressure of the fluid Is monitored, possibly by a pressure gauge Inside the balloon. Alternatively to measuring the pressure, the volume of the fluid being pumped to the volume may be monitored, either when being pumped to the balloon or after having been pumped to the balloon. The inflation of the balloon constitutes a mechanical stimulus of the hollow system.

For stimulating the hollow system thermally, the fluid may be heated or cooled to a chosen temperature before being pumped to the balloon. By monitoring the temperature of the fluid being pumped to the balloon, either before entering the balloon, or preferably after having entered the balloon, the thermal stimulus to the wall of the hollow system is known. For obtaining an even more precise measurement of the temperature, which the wall of the hollow system is subjected to, it will also be possible in stead or additionally, to monitor the temperature of the outer surface of the balloon, said outer surface being the part of the catheter being in physical contact with the inner side of the wall of the hollow system.

In a preferred embodiment of the apparatus, the catheter is provided with at least two canals for establishing a flow of fluid to and a corresponding flow of fluid from the balloon, By continuously flowing the fluid to and from the balloon, and by constantly monitoring the temperature of the fluid, it is assured that the temperature of the fluid in the balloon Is constant and do not change, even under Influence of any heating or cooling effected by the hollow system, the wall of which the balloon is abutting.

For stimulating the hollow system electrically, different kinds of electrical stimulation may take place such as electrodes 5 being attached to the outer surface of the balloon. In the situation shown in fig. 1, the electrical stimulus may have an amperage of up to 80 mA and a voltage of between 50 V and 100 V. In the situation shown, the apparatus is inserted in a hollow system being near the heart of the patient. Therefore, care must be taken when applying the electrical current. The electrical current is passed from an external source (not shown) for generating the current, through wires inside canals in the catheter and to one or more small holes 6 provided just above the balloon 4.

From there, the wires 7 are passed externally to the outside surface of the balloon, the surface abutting the wall of the hollow system. In the embodiment shown, a couple of electrodes 5 are attached to the outer surface of the balloon in order to pass the electrical current from the balloon to the wall of the hollow system.

For stimulating the hollow system chemically, a number of holes 8 may be provided in the catheter 1. In the embodiment shown, only one hole 8 is provided. The hole is provided between the proximal end 2 (see fig. 1) of the catheter 1 and the balloon 4. Alternatively, or additionally, one or more holes may be provided between the balloon 4 and the distal end 3 of the catheter 1. Any kind of chemical substance, either a chemical substance being foreign to the hollow system or a chemical substance being familiar to the hollow system, may be passed trough canals in the catheter and into the hollow system through the hole leading from the canal to the hollows system. A familiar chemical substance may be a substance commonly present In the bodily hollow system being stimulated, such as an acid like HCl in the esophagus, or such as bile salts in the small intestine. A foreign chemical substance may be a pharmaceutical intended for diagnostics or treatment of diseases in the bodily system being measured, such as smooth muscles relaxants.

For performing measurements of a force applied by the wall of the bodily hollow system strain gauges 9 are attached to the catheter 1 at a position between the proximal end 2 (see fig. 1) and the balloon 4. After the catheter is inserted into the hollow system, and subsequent to the inflation of the balloon, the catheter and the balloon is fixed longitudinally in relation to the hollow system. At the proximal end of the catheter, as mentioned with reference to fig. 1, the catheter is fixed to the nose, to the cheek or to any other suitable location at the proximal end of the catheter. During stimulation, the hollow system such as esophagus may perform longitudinal movements as a result of the stimulation subjected to the patient. These longitudinal movements are indicative of the motor function of the hollow system and result in a corresponding longitudinal extension or relaxation of the balloon and of the catheter. The longitudinal extension or relaxation of the catheter may thus be measured by the strain gauges.

Inside the balloon, holes 10 are provided for passing fluid to and from the balloon. The holes 10 may also be used for passing measuring means such as temperature gauges and/or pressure gauges to the inside of the balloon for measuring the temperature and the pressure of the fluid inside the balloon. Also other measuring means such as piezo-electrical elements and the such to be used in connection with equipment of the apparatus only being assigned to, but not being attached to the catheter.

The catheter is provided with a number of canals running inside the catheter. Some of the canals are intended for passing stimulating means or measuring means from the proximal end of the catheter to a more distant end of the catheter, either at a position before the balloon, or at a position inside the balloon or a position after the balloon toward the distal end of the catheter.

Thus, canals may be provided for passing electrical wires for performing electrical stimuli, canals may be provided for passing a chemical substance for performing chemical stimuli, and canals may be provided for passing electrical wires for gauges and other recording means attached to the balloon, provided inside the balloon, or attached or provided elsewhere along the extension of the catheter. It will be possible to use one canal for several purposes, thus perhaps one canal for passing more or all of the electrical wires provided in connection with means for electrical stimuli and means for recording temperature, pressure or any other physical property in relation to using the apparatus for measuring.

In the embodiment shown, preferably especially two canals are provided, one canal for passing a fluid from the proximal end of the catheter to the balloon and another canal for passing the fluid from the balloon to the proximal end. The one canal lead to one of the holes 10 inside the balloon, and the other canal lead form the other holes 10 inside the balloon. By providing those two canals it is possible to continuously pass a fluid to and from the balloon. Thereby, it is possible to maintain a chosen temperature of the fluid, substantially independent of any heating or cooling of the fluid in the balloon from the wall of the hollow system, which the balloon is abutting. As mentioned, these two holes 10 may also be used to pass wires for any measuring means situated inside the balloon

With reference to the figures, it is important to notice that the catheter and the balloon only form part of the apparatus according to the invention, i.e. the apparatus does not consist of the catheter and the balloon, but the apparatus comprises the catheter and the balloon. Other parts of the apparatus may comprise any exterior equipment for generating any of the stimuli and any additional equipment for recording data, possibly provided by the gauges or other recording means attached to, connected to or in any other way assigned to the catheter and the balloon.

### EXAMPLE OF AN APPARATUS

### Subjects

Eleven healthy subjects, 7 males and 4 females, mean age 40 ± 10.4 years were included. None had any previous or current visceral diseases. Specifically they denied any chest pain, heartburn, dyspepsia or irritable bowel syndrome-like symptoms. They received no medication and had no somatic pain complains. The local Ethics Committee approved the protocol. The experiment was carried out at the Gastrointestinal Research Laboratory at Aalborg Hospital a short distance from the Intensive Care Unit, where personnel and equipment for rescue operations were available.

### Intubation of stimulation device

A probe designed for multimodal stimulation (TensioMed, Homslet, Denmark) with modifications performed at the Technical Department at Aalborg University included a bag for impedance planimetry (IP), temperature stimuli and electrodes for electrical stimuli. The subjects fasted for at least four hours before the experiment. After applying a small amount of local anaesthetic spray (Xylocaine, AstraZenica, Sweden) in the nose, the participants were intubated. The bag was carefully folded, lubricated and the probe was inserted through the nostrils. The bag was first inserted into the stomach and then retracted to identify the location of the lower esophageal sphincter as a zone of high resting pressure that decreased with swallowing. Then the bag was placed 8 cm proximal to the sphincter and the probe was taped to the nose. After intubation the subjects were asked to lie down with the bead tilted by 30 degrees. After 30 min of rest, the experiment was performed in that position.

### Sensory assessment

The assessment parameters were 1) quantitative sensation intensity, 2) qualitative sensation and 3) referred pain size.

The sensory intensity was assessed continuously using an electronic visual analogue scale (VAS) (Gatehouse A/S, Aalborg, Denmark). Sensory assessment on a VAS can be complex, as visceral pain is diffuse and difficult to characterize. Therefore, the patients were trained in assessment of sensation to deep pressure at the muscles on the right forearm several times before the visceral stimuli were given. Although still debated, most sensory afferents in the gut are probably polymodal and encode both non-painful and painful sensations. We therefore decided to use the scale for both non-painful and painful sensations. The intensities of the *non-painful* sensations were scored on the VAS up to 5, where the following descriptors were used to characterize the sensations: 1= vague perception of mild sensation; 2 = definite perception of mild sensation; 3 = vague perception of moderate sensation and 4 = definite perception of moderate perception. Five was the discomfort/ pain threshold. A qualitative scale was added to the non-painful intensity scores, as the subjects were asked to assign the feeling to one of the following seven sensations: pressure, burning/warm, stinging, colicky/cramping, fullness/nausea, cold, and others. The method and descriptors were chosen according to earlier studies using bag distension in the gut. For the *painful* sensations the patients used the scale from 5-10 anchored at 5 = discomfort/slight pain to 10 = unbearable pain, with anchor words selected from the intensity scale in the Danish version of the McGill Pain Questionnaire (MPQ)⁷. Accordingly, when the subject reported that the stimuli resulted in pain and/or severe discomfort (above 5 on the non-painful scale) they were asked to score the intensity from 5-10 on the VAS. The VAS has previously been demonstrated to be useful to assess painful stimuli to electrical current and distension in the stomach, small and large intestine After the experiment the McGill Pain Questionnaire was used to assess the painful sensations qualitatively.

### Referred pain

After the stimulation the patients were asked about referred pain and if present, the area was marked with a pen and transferred to a transparent paper. Later the area was digitized (ACECAD D900+ Digitizer, Taiwan) and the size calculated (Sigma-Scan, Jandel Scientific, Canada).

### Stimulation device and protocol

The stimulation protocol was composed of electrical stimuli followed by mechanical, cold and warm stimuli. During all stimuli autonomic reactions were monitored and the result was displayed on-screen using a Biopac MP100 system (Biopac Systems Inc., Santa Barbara, California) including sensors and recording system. Electrocardiogram was recorded using a multi-lead electrocardiography (ECG) cable record with the following leads: I, II, III, aVR, aVL and AVF. A pulsoxymeter was connected to one finger of the right hand, allowing oxygen saturation monitoring along with changes in pulse rate. Respiration was monitored using a chest belt, adjusted for in- and expiration movements.
1. *Electrical stimuli:* Two flexible silver-chloride stimulation electrodes (2 x 4 mm) were glued to the bag (Fig.1). The electrodes were connected to a computer-controlled constant-current stimulator (NoxiTest A/S, Aalborg, Denmark). The maximum intensity of the current was limited to 80 mA. Previous systems were capable of inducing atrial capturing when parts of the esophagus near the heart were stimulated Hence, to increase the distance between the electrodes and the heart, the electrodes were placed on the dorsal side of the bag, which was inflated with 10 ml of water corresponding to a diameter of 15 mm. This inflation was not felt by any of the subjects. The construction of the probe made twisting impossible and a mark on the catheter secured that the electrodes were placed at the dorsal site. Thus, the bipolar stimuli secured a maximal electrical field opposite to the heart with a distance of at least approximately 20 mm from the heart. Two other safety procedures were included in the protocol: 1) To give electrical stimuli a special computer should be activated first (and it was disconnected after the stimulation was completed) and 2) the wires connecting the patient to the electrical stimulator were removed after the electrical stimulation. Electrical stimuli were given as single or repeated bursts. "Single burst" stimuli were defined as five rectangular constant-current pulses with duration of 1ms at 200 Hz. "Repeated burst" stimuli were defined as five "single burst" stimuli delivered at 2 Hz. These stimulus sequences have previously been shown to be suitable for evoking pain in the esophagus stomach, duodenum and colon. The stimulus intensity was blinded for the subjects. The current intensity was gradually increased in steps of 0.5mA with an interval of 15s until the pain detection threshold was found. We used a protocol with pseudo-random sequences including lower intensity stimuli interspersed with the ascending stimulus intensities. Such series have proven to be valuable in our previous studies using both electrical and mechanical stimuli. Thus, intermittent sham stimuli with either no current or the same current as in the previous step were given to secure that the subject did not automatically increase the sensory rating. The sensory and pain detection thresholds, corresponding to 1 and 5 on the VAS, were found for the single and repeated stimuli. The subject rated the most intense of the train of the five repeated stimuli. For the repeated stimuli a stimulus-response function was made at baseline, where the current intensities corresponding with 1,3,5,6 and 7 on the VAS scale were found.
2. *Mechanical stimuli:* The bag contained a four-electrode impedance planimetry system as described previously. The electrodes were located inside a cylindrical bag on a 70-cm long probe with a diameter of 4.5 mm (Fig.1). Two outer ring electrodes for excitation were placed on the probe with an interelectrode distance of 38 mm. A constant alternating current of 100 µA at 5 kHz was delivered to the electrodes from a current generator (Gatehouse Medical A/S, Norresundby, Denmark). Two ring electrodes for detection of potential differences were placed 2 mm apart and midway between the excitation electrodes. The detection electrodes were connected to an impedance-measuring system. The cylindrical bag was 40 mm in length and was made of 35 µm, non-conducting polyurethane. It completely enclosed the electrodes and a side-hole used for measurement of pressure within the bag. The bag could be inflated with electrically conducting fluid (0.09% saline) through a pair of infusion channels each with a diameter of 2 mm. It could be inflated to a cross-sectional area (CSA) of approximately 2000 mm² (diameter equal to 50 mm) without stretching the wall of the bag. The infusion channels were connected to an infusion pump (Type 111, Ole Dich Instrument Makers Aps, Hvidovre, Denmark), that was able to fill or empty the bag continuously at varying flow rates. A safety valve was connected with the pump allowing the subjects to stop the infusion at any time. The system was calibrated before the probe was inserted in the esophagus. The CSA of the bag was measured from the impedance of the fluid inside the bag. Hence, when a current is induced in a uniform cylinder by two excitation electrodes, the voltage difference between the detection electrodes is related to the impedance of the fluid and thus the CSA of the bag. Details of the calculations have been described previously. All data were digitized and stored electronically for later display and processing on the computer system (Gatehouse Medical A/S, Norresundby, Denmark). A few test stimuli were done for preconditioning the tissue and to teach the subjects to score the sensation intensity. These were followed by three baseline distensions with a constant infusion rate of 25 ml/min until the subject reported pain (5 on the VAS). After these stimuli a stimulus-response function was made, where the volume, pressure and CSA was recorded at intensities corresponding with 1,3,5,6 and 7 on the VAS scale.
3. *Temperature stimuli:* Recirculating water was infused into the same bag as used for the mechanical stimuli (Fig.1). The infusion channels in the catheter were attached to a manual pump system where 50 ml of water was infused into one channel and simultaneously sucked out in the other channel with a speed of approximately 300 ml/min. A temperature probe (PR electronics, Roende, Denmark) monitored the water temperature inside the bag. The time elapsing from one temperature level to the next was 60 sec. First, the system was filled with 10 ml of water with the desired temperature. In the range of 25-40 °C, this could not be felt by any of the subjects, excluding the possibility for the distension to contribute to the sensation. Immediately after filling of the bag, 50 ml of water with the desired temperature was re-circulated without changing the volume or pressure in the bag. In pilot experiments it was found that during perfusion of the esophagus, temperatures inside the bag of approximately 5, 10, 15, 20, 25, 30, 35, 40, 45 and 50°C corresponded with water temperatures of 0, 5, 10, 16, 22, 30, 35, 44, 52 and 60 °C in the pump system. Similar to the electrical stimuli, we used a pseudo-random, blinded sequence with lower intensity stimuli interspersed with the ascending stimulus intensities. In each series the stimuli were given with expected intrabag temperatures of 10, 5, 15, 20, 30, 35, 40, 25 (sham) 45 and 50 °C, with 2-3 additional sham stimuli having the same temperature as the previous stimulus interposed randomly.

## Claims

1. An apparatus for stimulating walls of bodily hollow systems and for measuring a subsequent physical reaction, said apparatus comprising a catheter (1) being provided with an inflatable balloon (4) situated between a proximal end (2) and a distal end (3) of the catheter, the apparatus comprising means for passing an inflating fluid, preferably a liquid, from the proximal end to the balloon, the apparatus furthermore comprising means for introducing a chemical stimulus into the hollow system, said means for introducing the chemical stimulus being positioned between the proximal end and the distal end of the catheter, and the apparatus being provided with means for measuring at least one of the following physical properties of the balloon subsequently to applying the chemical stimulus: the volume of the balloon, the cross-sectional area of the balloon seen in a direction parallel to a longitudinal extension of the bodily hollow system, when the apparatus is introduced into the body, the diameter of the balloon in a plane perpendicular to a longitudinal extension of the bodily hollow system, when the apparatus is introduced into the body, the tension of the balloon, the strain of the balloon, the pressure of a fluid inside the balloon, and the temperature of a fluid inside the balloon.

2. An apparatus according to claim 1, wherein said apparatus furthermore comprises means for introducing a number of the following stimuli into the hollow system: mechanical stimulus, thermal stimulus and electric stimulus, and said means for introducing said number of stimuli being positioned between the proximal end and the distal end of the catheter.

3. An apparatus according to claim 1 or 2, said apparatus having provided inside the balloon a number of the following measuring means: pressure gauges, temperature gauges, visualizational recording means, means for recording flow of fluid.

4. An apparatus according to claim 2, where the apparatus further comprises means for establishing a flow of fluid, and said apparatus capable of passing the flowing fluid from an inlet at a proximal end of the apparatus, being exteriorly accessible when the apparatus is introduced into the body, through a number of canals (10) in the apparatus to the balloon, and said apparatus also being capable of passing the pressurised fluid from the balloon back to an outlet at the proximal end through a number of other canals.

5. An apparatus according to any of claims 1-4, where the means for introducing the chemical stimulus into the hollow system, is a number of canals (8) for passing the chemical substance from the proximal end to a number of outlets for passing the chemical substance into the bodily hollow system, said outlets being provided in the vicinity of the balloon.

6. An apparatus according to any of claims 2-5, where the apparatus further comprises means for passing an electrical current through the apparatus, and said apparatus having a number canals (6) inside the catheter for passing electrical wires (7) from the proximal end to positions on an outer surface of the balloon, and the positions on the surface of the balloon intended for abutting an inner wall of the hollow system, when the balloon is inflated.

7. An apparatus according to claim 6, where the wires are passed from the proximal end to a position of the catheter between the proximal end and the balloon, where the wires at said position are passed from the canals inside the catheter through wire outlets to the exterior of the catheter, and where the wires are passed from the outlets to an outer surface of the balloon.

8. An apparatus according to claim 1, said apparatus having a number of measuring means attached to the balloon, said measuring means being selected from the group of strain gauges, pressure gauges, temperature gauges, piezo-electrical gauges, electrodes, pH-recording means, and electromyographic (EMG) recording means.

9. An apparatus according to claim 1 or claim 8, said apparatus having a number of measuring means inserted into the balloon, said measuring means being selected from the group of pressure gauges, temperature gauges, ultrasonic measuring means, visualizational recording means, means for recording flow of fluid.

10. An apparatus according to any of claims 1, 8-9, said apparatus having a number of measuring means assigned to the catheter outside the balloon, said measuring means being selected from the group of ultrasonic measuring means, visualizational recording means, scanning means, means for recording a flow of fluid.

11. An apparatus according to any of claims 1, 8-10, said apparatus having assigned to the catheter a number of the following measuring means for measuring the sensation felt by the patient of any of the stimuli being subjected: automatically operated scales, manually operated scales and personal written or orally expressions.

12. An apparatus according to any of the claims 1-11, the apparatus further comprising a number of strain gauges attached to the part of the catheter not being provided with the balloon, said strain gauges intended for measuring a longitudinal extension of the hollow system, and said strain gauges being applied to the catheter at a position between the proximal end and the balloon.

13. Apparatus according to any of claims 1-12 adapted for use in a part of the digestive system including the stomach and the gastrointestinal tract, the urogenital system including the urinary bladder, the cardiovascular system including the heart.

## Patentansprüche

1. Gerät zum Stimulieren von Wänden von körperlichen Hohlraumsystemen und zum Messen einer nachfolgenden physischen Reaktion, wobei das Gerät einen Katheter (1) umfasst, der mit einem aufblasbaren Ballon (4), der sich zwischen einem proximalen Ende (2) und einem distalen Ende (3) des Katheters befindet, ausgerüstet ist, und das Gerät Mittel zum Leiten eines Füllfluids, vorzugsweise einer Flüssigkeit, vom proximalen Ende zum Ballon umfasst, und das Gerät weiter Mittel zum Einführen eines chemischen Reizmittels in das Hohlraumsystem umfasst, wobei die Mittel zum Einführen des chemischen Reizmittels zwischen dem proximalen Ende und dem distalen Ende des Katheters positioniert sind, und das Gerät zur nachfolgenden Einführung des chemischen Reizmittels mit Mitteln zum Messen von mindestens einem der folgenden physischen Merkmale des Ballons ausgerüstet ist: das Volumen des Ballons, die Querschnittsfläche des Ballons, in einer Richtung parallel zu einer länglichen Erweiterung des körperlichen Hohlraumsystems gesehen, wenn das Gerät in den Körper eingeführt ist, der Durchmesser des Ballons in einer Ebene senkrecht zu einer länglichen Erweiterung des körperlichen Hohlraumsystems, wenn das Gerät in den Körper eingeführt ist, die Spannung des Ballons, die Dehnbeanspruchung des Ballons, der Druck eines Fluids innen im Ballon und die Temperatur eines Fluids innen im Ballon.

2. Gerät nach Anspruch 1, worin das Gerät weiter Mittel zum Einführen einer Anzahl der folgenden Reizmittel in das Hohlraumsystem umfasst: mechanisches Reizmittel, thermisches Reizmittel und elektrisches Reizmittel, und die Mittel zum Einführen der Anzahl von Reizmitteln, die zwischen dem proximalen Ende und dem distalen Ende des Katheters positioniert sind.

3. Gerät nach Anspruch 1 oder 2, wo das Gerät innen im Ballon mit einer Anzahl der folgenden Messmittel versehen ist: Drucksensoren, Temperatursensoren, Visualisierungserfassungsgeräte, Geräte zum Erfassen von Fluiddurchströmung.

4. Gerät nach Anspruch 2, wo das Gerät weiter Mittel zur Herstellung einer Fluiddurchströmung umfasst, und das Gerät imstande ist, das strömende Fluid von einer Eintrittsöffnung an einem proximalen Ende des Apparats, welches von außen, wenn das Gerät in den Körper eingeführt ist, zugänglich ist, durch eine Anzahl von Kanälen (10) im Gerät zum Ballon zu leiten, und das Gerät auch imstande ist, das druckbeaufschlagte Fluid vom Ballon zurück zu einer Auslassöffnung am proximalen Ende durch eine Anzahl von anderen Kanälen zu leiten.

5. Gerät nach irgendeinem der Ansprüche 1-4, wo die Mittel zum Einführen des chemischen Reizmittels in das Hohlraumsystem eine Anzahl von Kanälen (8) ist zum Leiten der chemischen Substanz vom proximalen Ende zu einer Anzahl von Auslassöffnungen zum Leiten der chemischen Substanz in das körperliche Hohlraumsystem, wobei die Auslassöffnungen in der Umgebung des Ballons vorgesehen sind.

6. Gerät nach irgendeinem der Ansprüche 2-5, wo das Gerät weiter Mittel zum Leiten eines elektrischen Stroms durch das Gerät umfasst, und das Gerät eine Anzahl von Kanälen (6) innen im Katheter hat, um elektrische Kabel (7) vom proximalen Ende zu Positionen an einer Außenfläche des Ballons zu leiten, und die Positionen an der Oberfläche des Ballons dafür vorgesehen sind, an eine Innenwand des Hohlraumsystems anzugrenzen, wenn der Ballon aufgeblasen ist.

7. Gerät nach Anspruch 6, wo die Kabel vom proximalen Ende zu einer Position des Katheters zwischen dem proximalen Ende und dem Ballon geleitet sind, wobei die Kabel in der Position von den Kanälen innen im Katheter durch Kabelauslassöffnungen zur Außenseite des Katheters geleitet sind, und wobei die Kabel von den Auslassöffnungen zu einer Außenfläche des Ballons geleitet sind.

8. Gerät nach Anspruch 1, wobei das Gerät eine Anzahl von am Ballon angebrachten Messmitteln hat, die aus der Gruppe von Dehnungssensoren, Drucksensoren, Temperatursensoren, piezoelektrischen Sensoren, Elektroden, pH-Wert-Erfassungsmitteln und elektromyographischen (EMG) Erfassungsmitteln ausgewählt sind.

9. Gerät nach Anspruch 1 oder Anspruch 8, wobei das Gerät eine Anzahl von in die Ballons eingesetzte Messmittel hat, die aus der Gruppe von Drucksensoren, Temperatursensoren, Ultraschall-Messgeräten, Visualisierungserfassungsgeräten, Mitteln zur Erfassung von Fluiddurchströmung ausgewählt sind.

10. Gerät nach irgendeinem der Ansprüche 1, 8 und 9, wobei das Gerät eine Anzahl von Messmitteln hat, die am Katheter außerhalb des Ballons zugeordnet sind und aus der Gruppe von Ultraschall-Messgeräten, Visualisierungserfassungsgeräten, Scanmitteln, Mitteln zur Erfassung von Fluiddurchströmung ausgewählt sind.

11. Gerät nach irgendeinem der Ansprüche 1 und 8 bis 10, wobei das Gerät eine Anzahl der folgenden Messmittel hat, die am Katheter zugeordnet sind, zum Messen des durch den Patienten erlebten Empfindens nach Anwendung irgendeines der beanspruchten Reizmittel: automatisch betätigte Meßgeräte, manuell betätige Meßgeräte und persönliche geschriebene oder mündliche Äußerungen.

12. Gerät nach irgendeinem der Ansprüche 1-11, wobei das Gerät weiter eine Anzahl von Dehnungssensoren umfasst, die an dem Teil des Katheters, der nicht mit dem Ballon ausgestattet ist, angebracht sind, wobei die Dehnungssensoren zur Messung einer länglichen Erweiterung des Hohlraumsystems vorgesehen sind, und die Dehnungssensoren am Katheter an einer Position zwischen dem proximalen Ende und dem Ballon angebracht sind.

13. Gerät nach irgendeinem der Ansprüche 1-12, angepasst zur Anwendung in einem Teil des Verdauungssystems, einschließlich Magen und Magen-Darm-Trakt, des urogenitalen Systems, einschließlich Harnblase, und des Herz-Kreislauf-Systems, einschließlich Herz.

## Revendications

1. Appareil pour stimuler les parois de systèmes creux corporels et pour mesurer une réaction physique subséquente, ledit appareil comprenant un cathéter (1) qui est muni d'un ballonnet gonflable (4) situé entre une extrémité proximale (2) et une extrémité distale (3) du cathéter, l'appareil comprenant un moyen pour faire passer un fluide de gonflage, de préférence un liquide, depuis l'extrémité proximale jusqu'au ballonnet, l'appareil comprenant en outre un moyen pour introduire un stimulus chimique dans le système creux, ledit moyen pour introduire le stimulus chimique étant positionné entre l'extrémité proximale et l'extrémité distale du cathéter, et l'appareil étant muni d'un moyen pour mesurer au moins une des propriétés physiques suivantes du ballonnet suite à l'application du stimulus chimique : le volume du ballonnet, la superficie en coupe du ballonnet vue dans une direction parallèle à une extension longitudinale du système creux corporel, lorsque l'appareil est introduit dans le corps, le diamètre du ballonnet dans un plan perpendiculaire à une extension longitudinale du système creux corporel, lorsque l'appareil est introduit dans le corps, la tension du ballonnet, la déformation du ballonnet, la pression d'un fluide à l'intérieur du ballonnet et la température d'un fluide à l'intérieur du ballonnet.

2. Appareil selon la revendication 1, dans lequel ledit appareil comprend en outre un moyen pour introduire un certain nombre des stimuli suivants dans le système creux : stimulus mécanique, stimulus thermique et stimulus électrique, et ledit moyen pour introduire ledit certain nombre de stimuli étant positionné entre l'extrémité proximale et l'extrémité distale du cathéter.

3. Appareil selon la revendication 1 ou 2, ledit appareil ayant, prévus à l'intérieur du ballonnet, un certain nombre des moyens de mesure suivants : manomètres, capteurs de température, moyens d'enregistrement de visualisation, moyens pour enregistrer un écoulement de fluide.

4. Appareil selon la revendication 2, où l'appareil comprend en outre un moyen pour établir un écoulement de fluide, et ledit appareil étant capable de faire passer le fluide en circulation depuis une entrée à une extrémité proximale de l'appareil, qui est accessible depuis l'extérieur lorsque l'appareil est introduit dans le corps, à travers un certain nombre de canaux (10) dans l'appareil, jusqu'au ballonnet, et ledit appareil étant également capable de refaire passer le fluide sous pression depuis le ballonnet jusqu'à une sortie à l'extrémité proximale à travers un certain nombre d'autres canaux.

5. Appareil selon l'une quelconque des revendications 1-4, où le moyen pour introduire le stimulus chimique dans le système creux est un certain nombre de canaux (8) pour faire passer la substance chimique depuis l'extrémité proximale jusqu'à un certain nombre de sorties pour faire passer la substance chimique dans le système creux corporel, lesdites sorties étant prévues au voisinage du ballonnet.

6. Appareil selon l'une quelconque des revendications 2-5, où l'appareil comprend en outre un moyen pour faire passer un courant électrique à travers l'appareil, et ledit appareil ayant un certain nombre de canaux (6) à l'intérieur du cathéter pour faire passer des fils électriques (7) depuis l'extrémité proximale jusque des positions sur une surface extérieure du ballonnet, et les positions sur la surface du ballonnet étant destinées à venir buter contre une paroi interne du système creux, lorsque le ballonnet est gonflé.

7. Appareil selon la revendication 6, où les fils sont passés depuis l'extrémité proximale jusqu'à une position du cathéter entre l'extrémité proximale et le ballonnet, où les fils au niveau de ladite position sont passés depuis les canaux à l'intérieur du cathéter à travers des sorties de fils jusque l'extérieur du cathéter, et où les fils sont passés depuis les sorties jusqu'à une surface extérieure du ballonnet.

8. Appareil selon la revendication 1, ledit appareil ayant un certain nombre de moyens de mesure fixés au ballonnet, lesdits moyens de mesure étant choisis parmi le groupe des extensomètres, des manomètres, des capteurs de température, des capteurs piézoélectriques, des électrodes, des moyens d'enregistrement du pH et des moyens d'enregistrement électromyographiques (EMG).

9. Appareil selon la revendication 1 ou la revendication 8, ledit appareil ayant un certain nombre de moyens de mesure insérés dans le ballonnet, lesdits moyens de mesure étant choisis parmi le groupe des manomètres, des capteurs de température, des moyens de mesure par ultrasons, des moyens d'enregistrement de visualisation, des moyens pour enregistrer un écoulement de fluide.

10. Appareil selon l'une quelconque des revendications 1, 8-9, ledit appareil ayant un certain nombre de moyens de mesure affectés au cathéter à l'extérieur du ballonnet, lesdits moyens de mesure étant choisis parmi le groupe des moyens de mesure par ultrasons, des moyens d'enregistrement de visualisation, des moyens de balayage, des moyens pour enregistrer un écoulement de fluide.

11. Appareil selon l'une quelconque des revendications 1, 8-10, ledit appareil ayant un certain nombre des moyens de mesure suivants, affectés au cathéter, pour mesurer la sensation ressentie par le patient de l'un des stimuli qui sont soumis : échelles à commande automatique, échelles à commande manuelle et expressions personnelles écrites ou orales.

12. Appareil selon l'une quelconque des revendications 1-11, l'appareil comprenant en outre un certain nombre d'extensomètres fixés à la partie du cathéter qui n'est pas munie du ballonnet, lesdits extensomètres étant destinés à mesurer une extension longitudinale du système creux, et lesdits extensomètres étant appliqués au cathéter à une position entre l'extrémité proximale et le ballonnet.

13. Appareil selon l'une quelconque des revendications 1-12, adapté pour être utilisé dans une partie du système digestif y compris l'estomac et le tractus gastro-intestinal, du système urogénital, y compris la vessie, du système cardiovasculaire, y compris le coeur.
